(19) **Europäisches Patentamt / European Patent Office / Office européen des brevets**

(11) **EP 3 177 736 B1**

(12) **FASCICULE DE BREVET EUROPEEN**

(45) Date de publication et mention de la délivrance du brevet:
**25.09.2019 Bulletin 2019/39**

(21) Numéro de dépôt: **15759895.4**

(22) Date de dépôt: **06.08.2015**

(51) Int Cl.:
***C12Q 1/6883*** (2018.01)

(86) Numéro de dépôt international:
**PCT/FR2015/052172**

(87) Numéro de publication internationale:
**WO 2016/020626 (11.02.2016 Gazette 2016/06)**

(54) **MICROARNS CARACTÉRISANT L'ACNÉ ET LEURS UTILISATIONS**

MIKRO-RNAS ZUR CHARAKTERISIERUNG VON AKNE UND VERWENDUNGEN DAVON

MICRORNAS CHARACTERISING ACNE AND THE USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.08.2014 FR 1457669**

(43) Date de publication de la demande:
**14.06.2017 Bulletin 2017/24**

(73) Titulaire: **Galderma Research & Development 06410 Biot (FR)**

(72) Inventeurs:
• **MOUNIER, Carine**
**F-06560 Valbonne (FR)**
• **DERET, Sophie**
**F-06250 Mougins (FR)**

(74) Mandataire: **Nederlandsch Octrooibureau P.O. Box 29720 2502 LS The Hague (NL)**

(56) Documents cités:
**WO-A2-2005/118806 WO-A2-2011/053257 US-A1- 2012 259 000**

• **Genechip: "Data Sheet GeneChip TM miRNA 3.0 Array", , 29 mars 2012 (2012-03-29), XP055222758, Extrait de l'Internet: URL:http://www.carrerasresearch.org/genech ip-mirna-3-0-array_38713.pdf [extrait le 2015-10-21]**

EP 3 177 736 B1

## Description

La présente invention relève du domaine de la médecine, et plus particulièrement du diagnostic et du traitement de l'acné.

## ART ANTERIEUR

L'acné est une pathologie multifactorielle fréquente qui atteint la peau riche en glandes sébacées (visage, région scapulaire, bras et régions intertrigineuses). Elle est la plus fréquente des dermatoses. Les facteurs pathogéniques suivants jouent un rôle déterminant dans la constitution de l'acné : la prédisposition génétique, la surproduction de sébum (séborrhée), les androgènes, les troubles de la kératinisation folliculaire (comédogenèse) et la colonisation bactérienne et les facteurs inflammatoires.

Il existe plusieurs formes d'acné, ayant toutes en commun l'atteinte des follicules pilosébacés. On peut citer notamment, l'acné conglobata, l'acné chéloïde de la nuque, l'acné médicamenteuse, l'acné miliaire récidivante, l'acné nécrotique, l'acné neonatorum, l'acné prémenstruelle, l'acné professionnelle, l'acné sénile, l'acné solaire, et l'acné vulgaire.

L'acné vulgaire, appelée également acné juvénile polymorphe, est la plus courante. Elle comprend quatre stades, mais le passage par tous les stades n'est pas obligatoire :

- Le stade 1 correspond à l'acné comédonienne caractérisée par un grand nombre de comédons ouverts et/ou fermés, et de microkystes.
- Le stade 2, ou acné papulopustuleuse, est de gravité légère à modérée. Elle est caractérisée par la présence de comédons ouverts et/ou fermés, de microkystes, mais également de papules rouges et de pustules. Elle touche principalement le visage et laisse peu de cicatrices.
- Le stade 3, ou acné papulocomédonienne, est plus grave et s'étend au dos, au thorax et aux épaules. Elle est accompagnée d'un plus grand nombre de cicatrices.
- Le stade 4, ou acné nodulokystique, s'accompagne de nombreuses cicatrices. Elle présente des nodules ainsi que des pustules volumineuses violacées et douloureuses.

Les différentes formes d'acné décrites précédemment peuvent être traitées par des actifs tels que les anti-séborrhéiques et les anti-infectieux, par exemple le peroxyde de benzoyle (notamment le produit Eclaran® commercialisé par la société Pierre Fabre), par des rétinoïdes tels que la trétinoïne (notamment le produit Retacnyl® commercialisé par la société Galderma) ou l'isotrétinoïne (produit Roaccutane® commercialisé par les Laboratoires Roche), ou par des dérivés d'acide naphtoïque. Les dérivés d'acide naphtoïque tels que notamment l'acide 6-[3-(1-adamantyl)-4-méthoxyphényl]-2-naphtoïque), communément appelé adapalène (produit Differine® commercialisé par la société Galderma), sont largement décrits et reconnus comme des principes actifs aussi efficaces que la trétinoïne pour le traitement de l'acné.

La combinaison de plusieurs traitements locaux (antibiotiques, rétinoides, peroxydes, zinc) est également utilisée en dermatologie pour permettre d'augmenter l'efficacité des principes actifs et de diminuer leur toxicité (Cunliffe W.J., J. Dermatol. Treat., 2000, 11 (suppl2), S13-S14).

L'application multiple de différents produits dermatologiques peut être assez lourde et astreignante pour le patient.

Ainsi, tout nouveau moyen de diagnostic ou pronostic et tout nouvel outil de criblage ou d'analyse de populations de patients est extrêmement utile.

## RESUME DE L'INVENTION

La présente invention est décrite dans les revendications ci-jointes. Elle est basée sur l'identification de microARNs qui sont différentiellement exprimés chez les sujets souffrant d'acné.

L'invention vise plus précisément l'ensemble des microARNs suivants : hsa-miR-223, hsa-miR-21*, hsa-miR-212, hsa-miR-21, hsa-miR-421, hsa-miR-29b-1*, hsa-miR-146b-5p, hsa-miR-146b-3p, hsa-miR-941, hsa-miR-27a*, et hsa-miR-155, comme biomarqueurs de l'acné.

Ces microARNs sont utiles comme biomarqueurs pour le diagnostic de l'acné, pour le criblage de molécules susceptibles d'être utilisées pour le traitement de l'acné, ou pour déterminer l'efficacité d'un traitement de l'acné.

La présente invention fournit une méthode pour diagnostiquer l'acné chez un sujet, comprenant la détermination dans un échantillon de derme et/ou d'épiderme dudit sujet de l'expression de l'ensemble des microARNs suivants : hsa-miR-223, hsa-miR-21*, hsa-miR-212, hsa-miR-21, hsa-miR-421, hsa-miR-29b-1*, hsa-miR-146b-5p, hsa-miR-146b-3p, hsa-miR-941, hsa-miR-27a*, et hsa-miR-155.

## DESCRIPTION DE L'INVENTION

*Définitions*

*MicroARNs*

Les microARNs sont de petits ARNs non codants de 18 à 25 nucléotides, exprimés chez la plupart des organismes eucaryotes, et qui jouent un rôle important dans la régulation de l'expression des gènes. Ce sont de puissants répresseurs post-transcriptionnels : en se fixant par complémentarité à des séquences nucléotidiques spécifiques présentes sur des ARNm, ils vont ainsi empêcher la traduction en protéines de ces transcrits dits « cibles ». Un microARN possède plusieurs ARNm cibles et réciproquement, un ARNm est la cible de plusieurs microARNs.

Tous les microARNs connus sont référencés dans la base miRBase (miRBase, http://www.mirbase.org). Des microARNs homologues peuvent être retrouvés dans plusieurs organismes ; un système d'annotation a donc été mis en place pour leur attribuer un identifiant unique. Les microARNs sont identifiés par un numéro précédé de l'abréviation « miR » ou « mir », qui permet une distinction entre le microARN mature (miR) et la structure tige-boucle du précurseur de microARN (mir). Un préfixe est utilisé pour distinguer les espèces comme par exemple hsa-miR-101 et mmu-miR-101 pour faire une différence entre le microARN chez l'Homme (hsa : *Homo sapiens*) et chez la Souris (mmu : *Mus musculus*). Dans certains cas, un même précurseur tige-boucle peut donner lieu à la synthèse de deux microARNs différents. Le système d'annotation permet la distinction de ces deux microARNs : le microARN du côté 5' de la boucle est noté 5p et le microARN du côté 3' est noté 3p. Cette annotation 3p et 5p est utilisée jusqu'à ce que l'abondance de l'une des deux formes soit déterminée. La forme majoritaire « miR-xxx » se distingue alors de la forme minoritaire « miR-xxx\* ».

Les microARNs sont impliqués dans une large gamme de processus biologiques clés tels que le contrôle du cycle cellulaire et l'apoptose. Ils régulent également plusieurs processus physiologiques et de développement comme la différenciation des cellules souches, l'hématopoïèse, l'hypoxie, le développement musculaire, la neurogenèse, la sécrétion d'insuline, le métabolisme du cholestérol, le vieillissement, les réponses immunitaires et inflammatoires. En outre, des schémas distincts d'expression temporelle au cours de l'embryogenèse ainsi que des profils d'expression spécifiques par tissu suggèrent que les microARNs jouent un rôle essentiel dans la différenciation et le maintien de l'identité des tissus. Ces processus biologiques sont souvent déréglés chez des individus malades. De nombreuses études ont mis en évidence l'implication des microARNs dans certaines pathologies, dont différents cancers, des maladies cardiaques et des maladies neurologiques. Plus récemment, le rôle des microARNs a également été démontré dans certaines maladies dermatologiques telles que le psoriasis, le vitiligo ou encore certains cancers de la peau.

*Sujets*

Les sujets sont des patients humains, homme ou femme, de tout âge.

*Echantillons biologiques*

Les échantillons testés sont typiquement des échantillons de derme, d'épiderme ou de derme et d'épiderme. Dans certains modes de réalisation, il s'agit d'une biopsie de peau, de préférence prélevées au niveau des zones de la peau atteintes. Plus spécifiquement, il s'agit de préférence de biopsies cutanées au niveau des zones lésionnelles, par exemple porteuses de papules.

Il peut aussi s'agir de tout échantillon de liquide biologique, comme du sang, de la salive, de l'urine.

*Contrôles*

Les échantillons « contrôles », ou des niveaux d'expression « contrôles », sont des échantillons, ou des niveaux d'expression, issus de sujets sains ou de sujets atteints d'une autre pathologie, éventuellement une autre pathologie cutanée.

*Applications diagnostiques*

La présente invention est relative à l'identification de microARNs et/ou de précurseurs de ceux-ci qui sont différentiellement exprimés chez les sujets souffrant d'acné par rapport à des sujets sains ou par rapport à des sujets atteints d'une autre pathologie cutanée, comme la rosacée, notamment la rosacée de type II.

Sur la base des microARNs identifiés, il est possible de :

- utiliser ces microARNs comme biomarqueurs de l'acné ;
- utiliser d'un ou plusieurs des microARNs identifiés pour le diagnostic, la détection, la détermination du stade, le suivie et le pronostic de l'acné ;
- réaliser une méthode de diagnostic, de détection, de détermination du stade, de suivi et de pronostic de l'acné, la méthode comprenant au moins une étape de détermination du niveau d'expression d'un ou plusieurs des microARNs identifiés dans un échantillon d'un patient.
- Préparer un kit de diagnostic, de détection, de détermination du stade, de suivi et de pronostic de l'acné, le kit comprenant des moyens de détection du niveau d'expression d'un ou plusieurs des microARNs identifiés. De préférence, les moyens de détection sont des acides nucléiques ou des peptides présentant une capacité à se lier à un ou plusieurs des microARNs identifiés, de préférence des oligonucléotides ou sondes spécifiques d'un ou plusieurs des microARNs identifiés ;
- utiliser ces microARNs pour effectuer des analyses de populations ou des clusterings, par exemple pour différentier les sujets souffrant de l'acné et les sujets sains.

Une fois le diagnostic posé, un traitement de l'acné peut être envisagé.

La méthode pour diagnostiquer l'acné chez un sujet selon l'invention comprend la détermination dans un échantillon dudit sujet de l'expression de l'ensemble des microARNs suivants : hsa-miR-223, hsa-miR-21*, hsa-miR-212, hsa-miR-21, hsa-miR-421, hsa-miR-29b-1*, hsa-miR-146b-5p, hsa-miR-146b-3p, hsa-miR-941, hsa-miR-27a*, et hsa-miR-155.

Avantageusement, une augmentation de l'expression de ces microARNs dans l'échantillon par rapport aux contrôles indique que le sujet souffre ou est susceptible de souffrir d'acné.

Dans un mode de réalisation particulier, la méthode comprend en outre la détermination supplémentaire dans un échantillon dudit sujet de l'expression d'au moins un des microARNs choisis dans le groupe consistant en hsa-miR-617, hsa-miR-885-5p, hsa-miR-652, hsa-miR-1281, hsa-miR-4310, hsa-miR-4800-3p, hsa-miR-4727-3p, hsa-miR-4791, hsa-miR-505*, hsa-miR-3651, hsa-miR-543, hsa-miR-23a*, hsa-miR-18a, hsa-miR-1246, hsa-miR-3615, hsa-miR-4484, hsa-miR-4800-5p, hsa-miR-629*, hsa-miR-30e*, hsa-miR-24-2*, hsa-miR-18b, hsa-miR-4667-5p, hsa-miR-483-5p, hsa-miR-27b*, hsa-miR-629, hsa-miR-187, hsa-miR-487a, hsa-miR-1244, hsa-miR-132, hsa-miR-550a*, hsa-miR-4286, hsa-miR-542-5p, hsa-let-7i*, hsa-miR-625, hsa-miR-411, hsa-miR-3175, hsa-miR-192, hsa-miR-503, hsa-miR-31, hsa-miR-1301 hsa-miR-92a-1*, hsa-miR-501-5p, hsa-miR-4732-5p, hsa-miR-150, hsa-miR-424*, hsa-miR-4417, hsa-miR-4521, hsa-miR-365*, et hsa-miR-181a*.

Selon un aspect préféré, la méthode comprend la détermination supplémentaire dans un échantillon dudit sujet de l'expression d'un ou plusieurs microARNs choisis dans le groupe consistant en hsa-miR-617, hsa-miR-885-5p, hsa-miR-652, hsa-miR-1281, hsa-miR-4310, hsa-miR-4800-3p, hsa-miR-4727-3p et hsa-miR-4791, dans laquelle une diminution de l'expression d'un ou plusieurs de ces microARNs dans l'échantillon par rapport aux contrôles indique que le sujet souffre ou est susceptible de souffrir d'acné.

Selon un autre aspect, la méthode comprend la détermination supplémentaire dans un échantillon dudit sujet de l'expression d'un ou plusieurs microARNs choisis dans le groupe consistant en hsa-miR-505*, hsa-miR-3651, hsa-miR-543, hsa-miR-23a*, hsa-miR-18a, hsa-miR-1246, hsa-miR-3615, hsa-miR-4484, hsa-miR-4800-5p, hsa-miR-629*, hsa-miR-30e*, hsa-miR-24-2*, hsa-miR-18b, hsa-miR-4667-5p, hsa-miR-483-5p, hsa-miR-27b*, hsa-miR-629, hsa-miR-187, hsa-miR-487a, hsa-miR-1244, hsa-miR-132, hsa-miR-550a*, hsa-miR-4286, hsa-miR-542-5p, hsa-let-7i*, hsa-miR-625, hsa-miR-411, hsa-miR-3175, hsa-miR-192, hsa-miR-503, hsa-miR-31, hsa-miR-1301 hsa-miR-92a-1*, hsa-miR-501-5p, hsa-miR-4732-5p, hsa-miR-150, hsa-miR-424*, hsa-miR-4417, hsa-miR-4521, hsa-miR-365*, hsa-miR-181a*, hsa-miR-155, hsa-miR-27a*, hsa-miR-941, hsa-miR-146b-3p, hsa-miR-146b-5p, hsa-miR-29b-1*, hsa-miR-421, hsa-miR-21, hsa-miR-212, hsa-miR-21*, et hsa-miR-223,

Il est également décrit un kit comprenant des moyens de détection spécifiques des microARNs des microARNs suivants : hsa-miR-223, hsa-miR-21*, hsa-miR-212, hsa-miR-21, hsa-miR-421, hsa-miR-29b-1*, hsa-miR-146b-5p, hsa-miR-146b-3p, hsa-miR-941, hsa-miR-27a*, et hsa-miR-155, de préférence des sondes ou amorces spécifiques.

La détection ou la quantification du ou des échantillons biologiques du ou des sujets contrôles peuvent être concomitantes à celles effectuées pour l'échantillon du patient ou provenir de données collectées antérieurement et disponibles, par exemple, sur une base de données.

Dans les méthodes selon l'invention, l'expression des microARNs peut être détectée ou quantifiée selon des méthodes bien connues de l'homme du métier, par exemple par RT-PCR quantitative et/ou par des techniques d'hybridation, par exemple à l'aide d'une sonde marquée ou par l'utilisation d'une puce.

Sur la base des microARNs identifiés, il est en outre possible de réaliser une méthode de suivi de l'efficacité thérapeutique d'un traitement dans laquelle une étape de détermination du niveau d'expression d'un ou plusieurs des microARNs identifiés dans un échantillon d'un patient est réalisée avant, pendant et/ou après traitement, et les niveaux d'expression sont comparés.

*Applications thérapeutiques*

Il est également possible de cibler la régulation de ces microARNs pour traiter ou prévenir l'acné ; la molécule utilisée pour le traitement est une molécule permettant de diminuer ou de supprimer la dérégulation de l'expression d'un ou plusieurs microARNs différentiellement exprimés chez les sujets souffrant d'acné. Notamment, les microARNs sous-exprimés chez les sujets souffrant d'acné pourraient être utilisés pour le traitement de l'acné par administration de ceux-ci. Alternativement, lorsque des microARNs sont sur-exprimés chez les sujets souffrant d'acné, le traitement visera à permettre l'augmentation de l'expression du gène cible, par exemple en bloquant l'effet de ces microARNs.

Il est décrit ici une composition pharmaceutique comprenant un ou plusieurs microARNs identifiés ou précurseurs de ceux-ci et son utilisation pour le traitement de l'acné ou la préparation d'un médicament destiné au traitement de l'acné. De préférence, la composition comprend en outre un ou plusieurs excipients et/ou véhicules pharmaceutiquement acceptables. Il est également décrit un vecteur d'expression comprenant une séquence nucléotidique codant pour un ou plusieurs des microARNs identifiés ou un précurseurs de ceux-ci et son utilisation pour le traitement de l'acné ou la préparation d'un médicament destiné au traitement de l'acné. De préférence, les microARNs identifiés sont choisis parmi les microARNs sous-exprimés chez les patients souffrant de l'acné. De préférence, la composition pharmaceutique est une composition dermatologique. En particulier, la composition est destinée à une administration topique.

Il est décrit aussi l'utilisation dans le traitement de l'acné d'une molécule ou combinaison de molécules, ladite molécule ou combinaison de molécules permettant d'augmenter ou de diminuer l'expression ou l'activité d'un ou plusieurs microARNs décrits ici.

Selon un aspect particulier, ladite molécule ou combinaison de molécules est un ou plusieurs microARNs décrits ici, ou précurseurs de ces microARNs, ou est un acide nucléique codant pour ledit ou lesdits microARNs ou précurseurs de ceux-ci, ou est un analogue, dérivé ou forme modifiée du ou des microARNs conservant son ou leur activité.

Selon un autre aspect, la molécule ou combinaison de molécules est un inhibiteur du ou des microARNs, de préférence un oligonucléotide sens ou antisens capable de s'hybrider au(x)dit(s) microARNs, inhibant ainsi la production et/ou l'activité du ou des microARNs ou augmentant la déplétion du ou des microARNs.

Il est également envisagé d'effectuer un criblage pour identifier de nouveaux médicaments pour le traitement de l'acné, dans lequel les molécules candidates seront testées quant à leur capacité de restaurer totalement ou partiellement une expression des microARNs dérégulés chez les sujets souffrant de l'acné et les molécules ayant l'effet souhaité seront sélectionnées.

Il est ainsi décrit une méthode de criblage de molécules utiles pour le traitement de l'acné, comprenant a) la mise en contact d'une cellule avec une molécule test, b) la détermination de l'expression d'un ou plusieurs microARNs tels que décrits ici et c) la sélection de la molécule test si elle augmente ou diminue l'expression ou l'activité d'un ou plusieurs desdits microARNs. Typiquement l'expression d'un microARN est analysée et comparée en présence et en absence de la molécule test.

La partie expérimentale qui suit illustre l'invention sans en limiter la portée.

## EXEMPLES

### *Introduction*

La présente invention est relative à l'identification de 60 microARNs et/ou de précurseurs de ceux-ci qui sont différentiellement exprimés chez les sujets souffrant d'acné par rapport à des sujets sains. Le miRNome a été caractérisé dans de la peau lésionnelle acnéique versus de la peau non lésionnelle. 11 sujets ont été étudiés avec pour chaque sujet, 1 biopsie dans le dos (peau non lésionnelle) et 1 biopsie dans le dos dans une zone lésionnelle (papule).

Afin de mener à bien la caractérisation du miRNome de la pathologie, une étude à large échelle a été menée, à l'aide des puces Affymetrix miRNA 3.0, afin d'établir les profils d'expression des microARNs.

### *Résultats*

Etude à large échelle des microARNs

La présente analyse a permis d'identifier 60 microARNs différentiellement exprimés dans la peau lésionnelle acnéique (LS) versus de la peau non lésionnelle (NLS).

**Tableau 1 : Liste des 60 microARNs matures modulés (|fold|≥1,5 et raw p-value≤0.05) dans l'analyse différentielle LS vs NLS (étude acné Blume : GRDS0051) à partir des 3391 identifiants Affymetrix**

| Affymetrix id | Fold Change | RawPValue | FDR_BH | mean expression LS | mean expression NLS |
|---|---|---|---|---|---|
| hsa-miR-617 | -2,1 | **2,4E-02** | 4,8E-01 | 6 | 13 |
| hsa-miR-885-5p | -1,8 | **4,7E-03** | 2,8E-01 | 15 | 28 |
| hsa-miR-652 | -1,7 | **2,0E-03** | 1,9E-01 | 456 | 756 |
| hsa-miR-1281 | -1,6 | **3,5E-02** | 5,4E-01 | 128 | 209 |
| hsa-miR-4310 | -1,6 | **2,0E-02** | 4,6E-01 | 5 | 9 |
| hsa-miR-4800-3p | -1,6 | **2,4E-02** | 4,8E-01 | 22 | 35 |
| hsa-miR-4727-3p | -1,6 | **3,3E-03** | 2,3E-01 | 4 | 6 |
| hsa-miR-4791 | -1,5 | **1,7E-02** | 4,3E-01 | 4 | 6 |
| hsa-miR-505* | 1,5 | **6,3E-03** | 2,9E-01 | 48 | 32 |
| hsa-miR-3651 | 1,6 | **4,3E-02** | 5,7E-01 | 50 | 32 |
| hsa-miR-543 | 1,6 | **5,7E-03** | 2,8E-01 | 4 | 2 |
| hsa-miR-23a* | 1,6 | **2,3E-03** | 1,9E-01 | 83 | 52 |
| hsa-miR-18a | 1,6 | **2,3E-03** | 1,9E-01 | 74 | 46 |
| hsa-miR-1246 | 1,6 | **1,9E-02** | 4,5E-01 | 148 | 92 |
| hsa-miR-3615 | 1,6 | **3,1E-02** | 5,0E-01 | 14 | 9 |
| hsa-miR-4484 | 1,6 | **1,3E-02** | 4,0E-01 | 166 | 102 |
| hsa-miR-4800-5p | 1,6 | **7,8E-03** | 3,3E-01 | 5 | 3 |
| hsa-miR-629* | 1,6 | **3,8E-02** | 5,6E-01 | 13 | 8 |
| hsa-miR-30e* | 1,7 | **3,5E-02** | 5,5E-01 | 9 | 5 |
| hsa-miR-24-2* | 1,7 | **1,9E-03** | 1,9E-01 | 52 | 31 |
| hsa-miR-18b | 1,7 | **1,9E-02** | 4,5E-01 | 6 | 3 |
| hsa-miR-4667-5p | 1,7 | **2,1E-02** | 4,6E-01 | 7 | 4 |
| hsa-miR-483-5p | 1,7 | **1,7E-02** | 4,3E-01 | 5 | 3 |
| hsa-miR-27b* | 1,7 | **8,3E-03** | 3,3E-01 | 53 | 31 |
| hsa-miR-629 | 1,7 | **8,0E-04** | 1,1E-01 | 45 | 26 |
| hsa-miR-187 | 1,7 | **4,0E-02** | 5,6E-01 | 21 | 12 |
| hsa-miR-487a | 1,7 | **1,8E-02** | 4,4E-01 | 6 | 3 |
| hsa-miR-1244 | 1,8 | **2,3E-02** | 4,7E-01 | 4 | 2 |
| hsa-miR-132 | 1,8 | **3,2E-05** | **1,4E-02** | 332 | 186 |
| hsa-miR-550a* | 1,8 | **6,0E-03** | 2,9E-01 | 9 | 5 |
| hsa-miR-4286 | 1,8 | **2,8E-02** | 4,8E-01 | 12 | 7 |
| hsa-miR-542-5p | 1,9 | **9,5E-03** | 3,4E-01 | 6 | 3 |
| hsa-let-7i* | 1,9 | **1,4E-02** | 4,1E-01 | 7 | 3 |
| hsa-miR-625 | 1,9 | **6,0E-03** | 2,9E-01 | 44 | 24 |
| hsa-miR-411 | 2,0 | **2,5E-02** | 4,8E-01 | 7 | 3 |
| hsa-miR-3175 | 2,0 | **4,4E-03** | 2,7E-01 | 10 | 5 |
| hsa-miR-192 | 2,0 | **2,8E-02** | 4,8E-01 | 12 | 6 |
| hsa-miR-503 | 2,0 | **2,2E-02** | 4,7E-01 | 8 | 4 |
| hsa-miR-31 | 2,1 | **6,2E-03** | 2,9E-01 | 445 | 215 |
| hsa-miR-1301 | 2,1 | **7,4E-03** | 3,2E-01 | 19 | 9 |
| hsa-miR-92a-1* | 2,1 | **5,1E-03** | 2,8E-01 | 24 | 12 |
| hsa-miR-501-5p | 2,2 | **6,0E-04** | 9,4E-02 | 18 | 8 |
| hsa-miR-4732-5p | 2,2 | **2,5E-03** | 1,9E-01 | 28 | 13 |
| hsa-miR-150 | 2,2 | **2,0E-04** | **4,9E-02** | 932 | 423 |
| hsa-miR-424* | 2,2 | **4,2E-03** | 2,7E-01 | 19 | 9 |
| hsa-miR-4417 | 2,3 | **1,7E-03** | 1,8E-01 | 24 | 10 |
| hsa-miR-4521 | 2,3 | **7,0E-05** | **1,8E-02** | 210 | 90 |

(suite)

| Affymetrix id | Fold Change | RawPValue | FDR_BH | mean expression LS | mean expression NLS |
|---|---|---|---|---|---|
| hsa-miR-365* | 2,4 | **4,0E-04** | 6,3E-02 | 27 | 11 |
| hsa-miR-181a* | 2,4 | **1,2E-03** | 1,5E-01 | 10 | 4 |
| hsa-miR-155 | 3,1 | **3,6E-07** | **4,0E-04** | 720 | 234 |
| hsa-miR-27a* | 3,1 | **6,0E-04** | 8,8E-02 | 52 | 17 |
| hsa-miR-941 | 3,2 | **5,3E-05** | **1,6E-02** | 14 | 4 |
| hsa-miR-146b-3p | 3,4 | **2,7E-05** | **1,3E-02** | 9 | 3 |
| hsa-miR-146b-5p | 3,7 | **1,0E-07** | **2,0E-04** | 208 | 56 |
| hsa-miR-29b-1* | 3,8 | **7,0E-05** | **1,8E-02** | 16 | 4 |
| hsa-miR-421 | 4,2 | **2,5E-05** | **1,3E-02** | 22 | 5 |
| hsa-miR-21 | 4,7 | **7,9E-05** | **1,9E-02** | 22 | 5 |
| hsa-miR-212 | 5,3 | **8,0E-07** | **7,0E-04** | 18 | 3 |
| hsa-miR-21* | 10,0 | **1,3E-07** | **2,0E-04** | 28 | 3 |
| hsa-miR-223 | 15,0 | **2,6E-06** | **1,8E-03** | 99 | 7 |

Note : Lorsque la valeur de FDR est surlignée en gras alors FDR ≤ 0,05

Un biomarqueur doit préférablement être fortement exprimé dans la pathologie et faiblement voire non exprimé chez les individus sains. En se basant à la fois sur le fold modulation (le plus élevé possible) et l'expression dans la peau saine (la plus faible possible), une liste de 11 biomarqueurs potentiels de l'acné a été établie. Cette liste comprend les microARNs : hsa-miR-223, hsa-miR-21*, hsa-miR-212, hsa-miR-21, hsa-miR-421, hsa-miR-29b-1*, hsa-miR-146b-5p, hsa-miR-146b-3p, hsa-miR-941, hsa-miR-27a*, et hsa-miR-155 (encadrés ci-dessus).

### *Matériel et méthodes*

Extraction des ARN totaux (ARNm et microARNs)

L'extraction des ARN totaux a été réalisée auparavant afin de réaliser l'étude du transcriptome. Cette extraction a été réalisée avec le kit miRNeasy Mini de QIAGEN qui permet d'extraire les grands ARNs (ARNm) ainsi que les petits ARNs incluant les microARNs. Ce kit comporte trois étapes successives : la lyse des tissus pour libérer les ARN contenus dans les cellules, l'isolation des ARNs sur membrane puis leur élution. Les résultats de l'étude transcriptomique précédemment établis ont été utilisés pour l'interprétation biologique des données sur les microARNs obtenues.

Etude à large échelle des microARNs

*Etude de l'expression des gènes de microARNs : puce Affymetrix miRN*

La puce miRNA 3.0 est composée des fragments d'acides nucléiques, appelés sondes, liées au support physique et dont l'ensemble des séquences correspond aux microARNs de 153 organismes dont plus de 1700 formes matures de microARNs humains. Chaque sonde (fragment d'ADN simple brin) est complémentaire d'un microARN donné. Pour chaque microARN, plusieurs sondes, variant parfois de quelques paires de bases, sont présentes sur la puce. C'est ce qu'on appelle un ensemble de sondes. La puce miRNA 3.0 contient près de 20 000 ensembles de sondes dont plus de 5 600 spécifiques aux petits ARNs humains dont les microARNs (1 733 matures et 1 658 précurseurs). Le design de cette puce a été réalisé à partir de miRBase version 17. Un ensemble de sondes dédié à un microARN mature est composé de 9 sondes identiques, complémentaires à la séquence de l'ARN mature cible.

L'expérience est faite à partir de 300 ng d'ARN totaux. La première étape consiste à ajouter une queue poly(A) aux microARNs. Cette queue poly(A), initialement présente sur les ARNm et dorénavant également sur les microARNs, va permettre la fixation de la molécule 3DNA® étiquetée avec 15 biotines. On aboutit alors à la formation d'ARN biotinylés. La deuxième étape est l'hybridation des ARN sur leurs sondes respectives ; les sondes présentes dans les différents compartiments de la puce étant spécifiques des microARNs, les ARNm biotinylés se retrouvent excluent tandis que les microARNs biotinylés vont s'hybrider aux sondes. Un lavage permet d'éliminer les molécules qui ne se sont pas hybridées sur la puce. Dans une troisième étape, la streptavidine couplée à une molécule fluorescente, la phycoerythrine est ajoutée. Grâce à son affinité pour la biotine, la streptavidine va permettre de mettre en évidence les microARNs biotinylés.

Afin d'optimiser ce marquage, une amplification du marquage est réalisée : pour cela, un anticorps de chèvre anti-streptavidine couplé à une biotine est introduit. Ce dernier va se fixer au complexe précédent microARN biotinylé-Streptavidine/phycoérythrine. L'ajout, une deuxième fois, de streptavidine/phycoerythrine, va avoir pour effet d'amplifier le marquage fluorescent afin d'améliorer la détection. Les étapes de marquage et lavages successifs sont automatisées grâce à une plateforme Affymetrix dédiée. Le signal fluorescent est ensuite traité au moyen du logiciel Expression console, fourni par Affymetrix. Plus un microARN est fixé en grande quantité sur sa sonde complémentaire, plus le signal lumineux sera intense. Ainsi, pour chaque puce, c'est en comparant l'intensité lumineuse correspondant à chaque sonde d'un échantillon traité versus un échantillon témoin que l'on peut analyser l'effet de ce traitement sur l'expression des gènes.

*Biostatistiques*

Sur un plan technique, les études de transcriptomique sont à la fois fiables et associées à des coûts abordables. Toutefois, l'analyse des quantités importantes de données générées demeure un point crucial. Dans ce domaine, le recours aux outils bioinformatiques et aux biostatistiques est indispensable. Il n'y a pas toujours de consensus concernant le choix des algorithmes mathématiques utilisés pour traiter les données. Pour les études du miRNome notamment, à large échelle ou à plus petite échelle par qPCR, plusieurs méthodes de normalisation des données brutes ont été proposées. Concernant les études à large échelle de l'expression des microARNs, la méthode la plus utilisée actuellement est la normalisation RMA. Avant normalisation, les données d'expression sont transformées en $\log_2$ afin d'aboutir à une gamme linéaire et de réduire l'étendue des données.

Logiciel Array studio : Array studio est un package logiciel conçu pour les biologistes et bioinformaticiens afin d'analyser statistiquement des données de séquençage nouvelle génération, de SNPs ou encore de microarray. Il inclut notamment le support pour toutes les plateformes microarray dont Agilent, Illumina et Affymetrix, cette dernière étant utilisée dans le cadre de notre étude. Il permet dans un premier temps de normaliser les données brutes selon diverses méthodes, puis dans un second temps de réaliser des analyses différentielles inter-groupes pour comparer les profils d'expression des microARNs.

Normalisation RMA (Robust Multiarray/Multichip Average) : Les études à large échelle rendent désormais possible l'analyse des profils d'expression de nombreux gènes en une seule expérience ; par exemple pour caractériser le miRNome d'un individu. Ces expériences, répétées pour chaque échantillon (individu) testé, génèrent beaucoup de données mais ces données peuvent être biaisées par des variations d'origine technologique. Le but de la normalisation est d'enlever le bruit lié à la technologie afin de prendre en compte au plus précisément les variations d'origine biologique. La normalisation RMA comporte trois étapes successives : la correction du bruit de fond ; la normalisation par quantiles : elle s'effectue sur l'ensemble des puces de l'étude considérée et tend à homogénéiser les distributions des niveaux d'expression entre les puces afin de les analyser conjointement, ou encore les comparer; et le « median polish » : étape au cours de laquelle les intensités de fluorescence des cibles se fixant sur un même ensemble de sondes sont combinées afin d'estimer une valeur d'intensité unique, par puce, pour chaque ensemble de cibles (ou gène).

Analyses différentielles

La caractérisation du miRNome dans une condition pathologique se fait par comparaison aux profils des microARNs obtenus dans une condition saine. Le principe de cette comparaison repose sur le calcul du « fold modulation » (modulation du niveau d'expression d'un gène). Pour connaître de degré de confiance accordé à ce fold, une p-value lui est associée, calculée grâce à un test d'hypothèses appelé test de Student.

*Calcul du « fold modulation »*

a) Expression relative des données à large échelle (Affymetrix)

Comme les données d'expression normalisées ont été transformées en $\log_2$, l'expression relative se calcule de la manière suivante :

$$\text{Expression relative} = 2^{\Delta}$$

avec $\Delta = \log_2(\text{Expression moyenne}_{\text{gène i condition B}}) - \log_2(\text{Expression moyenne}_{\text{gène i condition A/référence}})$

b) Facteur de modulation ou « Fold »

Le « fold modulation » se traduit par la modulation de la moyenne d'expression du gène i dans la condition A (de référence) par rapport à la condition B ; si le fold est positif alors le gène i est surexprimé dans la condition A par rapport à la condition B contrôle. A l'inverse, si le fold est négatif alors le gène i est sous-exprimé dans la condition A par rapport à la condition B contrôle. Dans le cas où la valeur d'expression relative calculée est supérieure à 1, elle équivaut à un facteur de modulation (« Fold ») positif. En revanche, dans le cas où l'expression relative calculée est comprise entre 0 et 1, signifiant que le gène est plus exprimé dans la condition de référence (A) que dans la condition testée (B), on réalise alors l'opération -1/fold pour obtenir des Folds négatifs, plus facilement interprétables. Un seuil sur le fold peut être fixé par le biologiste pour sélectionner spécifiquement ceux ayant un intérêt d'un point de vue biologique ; dans cette étude, il est fixé à +/-1,5.

*Principe d'un test d'hypothèses*

En statistique, un test d'hypothèses consiste à évaluer une hypothèse statistique en fonction de jeu de données (échantillon). Par exemple, si on pose la question : Est-ce que le gène *i* est différentiellement exprimé dans la condition A par rapport à la condition B ? La condition A peut correspondre à des individus malades et la condition B des individus sains contrôles.
Répondre par oui ou non à la question revient à trancher entre deux hypothèses :

- Hypothèse nulle ou H0 : le gène n'est pas différentiellement exprimé,
- Hypothèse alternative ou H1 : le gène est différentiellement exprimé.

Deux types d'erreurs peuvent se poser :

- Erreur de type I : rejeter H0 alors que H0 est vraie, revient à considérer que le gène est différentiellement exprimé alors qu'il ne l'est pas ; il s'agit alors d'un faux positif.
- Erreur de type II : ne pas rejeter H0 alors que H0 est fausse, revient à considérer que le gène n'est pas différentiellement exprimé alors qu'il l'est en réalité ; il s'agit dans ce cas d'un faux négatif.

Le risque maximum toléré (noté a) est appelée seuil de significativité ; il est généralement fixée à 0,05. Dans notre exemple, cela revient en moyenne à accepter le risque de se tromper 5 fois sur 100, et donc d'accepter en moyenne 5% de faux positifs dans notre analyse.
La statistique du test est calculée à partir des données. Sa valeur permet d'estimer le pourcentage de chance (p-value) nécessaire pour obtenir ces données si H0 est vraie. En fonction du seuil de significativité préalablement fixé et de cette p-value, la décision de rejeter ou non H0 peut être prise.

*Test de Student ou t-test*

Le test permet d'évaluer si les moyennes d'expression du gène i dans chaque condition A et B sont statistiquement différentes l'une de l'autre et ainsi de comparer une condition par rapport à une autre. La p-value du t-test permet ensuite de conclure si la différence entre les moyennes n'est pas due au hasard :

- Si p-value $\leq$ 0,05 alors on rejette H0 ; les moyennes des deux conditions A et B sont dîtes significativement différentes, le gène i est donc modulé de manière significative dans la condition A par rapport à la condition B.
- Si p-value > 0.05 alors on ne rejette pas H0 ; les moyennes des deux conditions A et B sont dîtes non-significativement différentes, le gène i n'est donc pas modulé de manière significative dans la condition A par rapport à la condition B.

*Contrôle du taux de fausses découvertes ou FDR*

Les puces d'expression des analyses à large échelle permettent de mesurer simultanément l'activité transcriptionnelle de plusieurs milliers de gènes pour un échantillon biologique donné. Pour les analyser de manière simultanée, on multiplie les tests d'hypothèses. Toutefois, lorsque l'on multiplie les tests, la probabilité de détecter un gène significativement modulé, alors qu'il ne l'est pas, augmente. Pour contrôler le taux de fausses découvertes (gènes considérés modulés alors qu'ils ne le sont pas), on contrôle le taux de fausses découvertes grâce à une méthode mise au point par Benjamini et Hochberg 1995, Journal of the Royal Statistical Society. Series B (Methodological) vol. 57, No. 1 (1995), pp. 289-300, le FDR (False Discovery Rate) qui corrige les p-values associées aux t-tests afin de conserver le même taux de fausses découvertes quel que soit le nombre de gènes. En conclusion, après une analyse différentielle, le gène *i* est considéré significativement modulé s'il respecte deux conditions :

- |fold| $\geq$ 1,5
- FDR < 0,05

**Revendications**

1. Méthode pour diagnostiquer l'acné chez un sujet, comprenant la détermination dans un échantillon de derme et/ou d'épiderme dudit sujet de l'expression de l'ensemble des microARNs suivants : hsa-miR-223, hsa-miR-21*, hsa-miR-212, hsa-miR-21, hsa-miR-421, hsa-miR-29b-1*, hsa-miR-146b-5p, hsa-miR-146b-3p, hsa-miR-941, hsa-miR-27a*, et hsa-miR-155, et dans laquelle une augmentation de l'expression desdits microARNs dans ledit échantillon par rapport à des échantillons contrôles indique que le sujet souffre d'acné ou est susceptible de souffrir d'acné.

2. Méthode selon la revendication 1, comprenant la détermination supplémentaire dans un échantillon dudit sujet de l'expression d'au moins un des microARNs choisis dans le groupe consistant en hsa-miR-617, hsa-miR-885-5p, hsa-miR-652, hsa-miR-1281, hsa-miR-4310, hsa-miR-4800-3p, hsa-miR-4727-3p, hsa-miR-4791, hsa-miR-505*, hsa-miR-3651, hsa-miR-543, hsa-miR-23a*, hsa-miR-18a, hsa-miR-1246, hsa-miR-3615, hsa-miR-4484, hsa-miR-4800-5p, hsa-miR-629*, hsa-miR-30e*, hsa-miR-24-2*, hsa-miR-18b, hsa-miR-4667-5p, hsa-miR-483-5p, hsa-miR-27b*, hsa-miR-629, hsa-miR-187, hsa-miR-487a, hsa-miR-1244, hsa-miR-132, hsa-miR-550a*, hsa-miR-4286, hsa-miR-542-5p, hsa-let-7i*, hsa-miR-625, hsa-miR-411, hsa-miR-3175, hsa-miR-192, hsa-miR-503, hsa-miR-31, hsa-miR-1301 hsa-miR-92a-1*, hsa-miR-501-5p, hsa-miR-4732-5p, hsa-miR-150, hsa-miR-424*, hsa-miR-4417, hsa-miR-4521, hsa-miR-365*, et hsa-miR-181a*.

3. Méthode selon la revendication 1, comprenant la détermination supplémentaire dans un échantillon dudit sujet de l'expression d'un ou plusieurs microARNs choisis dans le groupe consistant en hsa-miR-617, hsa-miR-885-5p, hsa-miR-652, hsa-miR-1281, hsa-miR-4310, hsa-miR-4800-3p, hsa-miR-4727-3p et hsa-miR-4791, dans laquelle une diminution de l'expression d'un ou plusieurs de ces microARNs dans l'échantillon par rapport aux contrôles indique que le sujet souffre ou est susceptible de souffrir d'acné.

4. Méthode selon la revendication 1, comprenant la détermination supplémentaire dans un échantillon dudit sujet de l'expression d'un ou plusieurs microARNs choisis dans le groupe consistant en hsa-miR-505*, hsa-miR-3651, hsa-miR-543, hsa-miR-23a*, hsa-miR-18a, hsa-miR-1246, hsa-miR-3615, hsa-miR-4484, hsa-miR-4800-5p, hsa-miR-629*, hsa-miR-30e*, hsa-miR-24-2*, hsa-miR-18b, hsa-miR-4667-5p, hsa-miR-483-5p, hsa-miR-27b*, hsa-miR-629, hsa-miR-187, hsa-miR-487a, hsa-miR-1244, hsa-miR-132, hsa-miR-550a*, hsa-miR-4286, hsa-miR-542-5p, hsa-let-7i*, hsa-miR-625, hsa-miR-411, hsa-miR-3175, hsa-miR-192, hsa-miR-503, hsa-miR-31, hsa-miR-1301 hsa-miR-92a-1*, hsa-miR-501-5p, hsa-miR-4732-5p, hsa-miR-150, hsa-miR-424*, hsa-miR-4417, hsa-miR-4521, hsa-miR-365*, hsa-miR-181a*, hsa-miR-155, hsa-miR-27a*, hsa-miR-941, hsa-miR-146b-3p, hsa-miR-146b-5p, hsa-miR-29b-1*, hsa-miR-421, hsa-miR-21, hsa-miR-212, hsa-miR-21*, et hsa-miR-223.

5. Utilisation de l'ensemble des microARNs suivants : hsa-miR-223, hsa-miR-21*, hsa-miR-212, hsa-miR-21, hsa-miR-421, hsa-miR-29b-1*, hsa-miR-146b-5p, hsa-miR-146b-3p, hsa-miR-941, hsa-miR-27a*, et hsa-miR-155, comme biomarqueurs de l'acné, par détection dans un échantillon de derme et/ou d'épiderme.

6. Méthode pour suivre ou déterminer l'efficacité d'un traitement de l'acné chez un sujet comprenant la détermination dans un échantillon de derme et/ou épiderme dudit sujet de l'expression des microARNs suivants : hsa-miR-223, hsa-miR-21*, hsa-miR-212, hsa-miR-21, hsa-miR-421, hsa-miR-29b-1*, hsa-miR-146b-5p, hsa-miR-146b-3p, hsa-miR-941, hsa-miR-27a*, et hsa-miR-155, et la diminution ou l'augmentation de l'expression desdits microARNs à l'issue ou pendant le traitement indiquant l'efficacité du traitement.

7. Méthode selon l'une des revendications 1-4, 6 et utilisation selon la revendication 5, dans laquelle l'échantillon est une biopsie de peau prélevée au niveau des zones de la peau atteintes.

**Patentansprüche**

1. Verfahren zur Diagnose der Akne bei einer Person, umfassend Ermittlung, in einer Lederhaut- und/oder Epidermisprobe der genannten Person, der Expression aller folgenden MikroRNAs: hsa-miR-223, hsa-miR-21*, hsa-miR-212, hsa-miR-21, hsa-miR-421, hsa-miR-29b-1*, hsa-miR-146b-5p, hsa-miR-146b-3p, hsa-miR-941, hsa-miR-27a* und hsa-miR-155, wobei eine Zunahme der Expression der genannten MikroRNAs in besagter Probe im Verhältnis zu Kontrollproben angibt, dass die Person für Akne anfällig ist oder an Akne leidet.

2. Verfahren nach Anspruch 1, umfassend zusätzlicher Ermittlung, in einer Probe der genannten Person, der Expression wenigstens einer der MikroRNAs aus der Gruppe hsa-miR-617, hsa-miR-885-5p, hsa-miR-652, hsa-miR-1281, hsa-miR-4310, hsa-miR-4800-3p, hsa-miR-4727-3p, hsa-miR-4791, hsa-miR-505*, hsa-miR-3651, hsa-miR-543, hsa-miR-23a*, hsa-miR-18a, hsa-miR-1246, hsa-miR-3615, hsa-miR-4484, hsa-miR-4800-5p, hsa-miR-629*, hsa-miR-30e*, hsa-miR-24-2*, hsa-miR-18b, hsa-miR-4667-5p, hsa-miR-483-5p, hsa-miR-27b*, hsa-miR-629, hsa-miR-187, hsa-miR-487a, hsa-miR-1244, hsa-miR-132, hsamiR-550a*, hsa-miR-4286, hsa-miR-542-5p, hsa-let-7i*, hsa-miR-625, hsa-miR-411, hsa-miR-3175, hsa-miR-192, hsa-miR-503, hsa-miR-31, hsa-miR-1301 hsa-miR-92a-1*, hsa-miR-501-5p,hsa-miR-4732-5p, hsa-miR-150, hsa-miR-424*, hsa-miR-4417, hsa-miR-4521, hsa-miR-365* und hsa-miR-181a*.

3. Verfahren nach Anspruch 1, umfassend zusätzlicher Ermittlung in einer Probe der genannten Person, der Expression einer oder mehrerer MikroRNAs aus der Gruppe hsa-miR-617, hsa-miR-885-5p, hsa-miR-652, hsa-miR-1281, hsa-miR-4310, hsamiR-4800-3p, hsa-miR-4727-3p und hsa-miR-4791, wobei eine Verringerung der Expression einer oder mehrerer diese MikroRNAs in der Probe im Vergleich zu den Kontrollproben angibt, dass die Person für Akne anfällig ist oder an Akne leidet.

4. Verfahren nach Anspruch 1, umfassend zusätzlicher Ermittlung, in einer Probe der genannten Person, der Expression einer oder mehrerer MikroRNAs aus der Gruppe hsa-miR-505*, hsa-miR-3651, hsa-miR-543, hsa-miR-23a*, hsa-miR-18a, hsamiR-1246, hsa-miR-3615, hsa-miR-4484, hsa-miR-4800-5p, hsa-miR-629*, hsa-miR-30e*, hsa-miR-24-2*, hsa-miR-18b, hsa-miR-4667-5p, hsa-miR-483-5p, hsa-miR-27b*, hsa-miR-629, hsa-miR-187, hsa-miR-487a, hsa-miR-1244, hsa-miR-132, hsa-miR-550a*, hsa-miR-4286, hsa-miR-542-5p, hsa-let-7i*, hsa-miR-625, hsamiR-411, hsa-miR-3175, hsa-miR-192, hsa-miR-503, hsa-miR-31, hsa-miR-1301 hsa-miR-92a-1*, hsa-miR-501-5p, hsa-miR-4732-5p, hsa-miR-150, hsa-miR-424*, hsa-miR-4417, hsa-miR-4521, hsa-miR-365*, hsa-miR-181a*, hsa-miR-155, hsa-miR-27a*, hsa-miR-941, hsa-miR-146b-3p, hsa-miR-146b-5p, hsa-miR-29b-1*, hsa-miR-421, hsa-miR-21, hsa-miR-212, hsa-miR-21* und hsa-miR-223.

5. Verwendung der folgenden MikroRNAs: hsa-miR-223, hsa-miR-21*, hsa-miR-212, hsa-miR-21, hsa-miR-421, hsa-miR-29b-1*, hsa-miR-146b-5p, hsa-miR-146b-3p, hsa-miR-941, hsa-miR-27a* und hsa-miR-155 als Biomarker für Akne, bei Entdeckung in einer Lederhaut- und/oder Epidermisprobe.

6. Verfahren, um die Effektivität einer Aknebehandlung bei einer Person zu verfolgen oder zu bestimmen, umfassend Ermittlung, in einer Lederhaut- und/oder Epidermisprobe der genannten Person, der Expression der folgenden MikroRNAs: hsa-miR-223, hsa-miR-21*, hsa-miR-212, hsa-miR-21, hsa-miR-421, hsa-miR-29b-1*, hsa-miR-146b-5p, hsa-miR-146b-3p, hsa-miR-941, hsa-miR-27a* und hsa-miR-155, wobei die Verringerung oder der Anstieg der Expression der genannten MikroRNAs am Ende oder während der Behandlung die Effektivität der Behandlung angibt.

7. Verfahren nach einem der Ansprüche 1-4, 6 und Verwendung nach Anspruch 5, wobei die Probe eine Biopsie von Haut ist, die in den Bereichen der erkrankten Haut entnommen wurde.

**Claims**

1. A method for diagnosing acne in a subject, comprising determining in a dermal and/or epidermal sample from said subject the expression of the following microRNAs: hsa-miR-223, hsa-miR-21*, hsa-miR-212, hsa-miR-21, hsa-miR-421, hsa-miR-29b-1*, hsa-miR-146b-5p, hsa-miR-146b-3p, hsa-miR-941, hsa-miR-27a*, and hsa-miR-155, and wherein an increase in the expression of said microRNAs in said sample compared to control samples indicates that the subject suffers from acne or is likely to suffer from acne.

2. The method of claim 1, comprising further determining in a sample from said subject the expression of at least one of the microRNAs selected from the group consisting of hsa-miR-617, hsa-miR-885-5p, hsa-miR-652, hsa-miR-1281, hsa-miR-4310, hsa-miR-4800-3p, hsa-miR-4727-3p, hsa-miR-4791, hsa-miR-505*, hsa-miR-3651, hsa-miR-543, hsa-miR-23a*, hsa-miR-18a, hsa-miR-1246, hsa-miR-3615, hsa-miR-4484, hsa-miR-4800-5p, hsa-miR-629*, hsa-miR-30e*, hsa-miR-24-2*, hsa-miR-18b, hsa-miR-4667-5p, hsa-miR-483-5p, hsa-miR-27b*, hsa-miR-629, hsa-miR-187, hsa-miR-487a, hsa-miR-1244, hsa-miR-132, hsamiR-550a*, hsa-miR-4286, hsa-miR-542-5p, hsa-let-7i*, hsa-miR-625, hsamiR-411, hsa-miR-3175, hsa-miR-192, hsa-miR-503, hsa-miR-31, hsa-miR-1301 hsa-miR-92a-1*, hsa-miR-501-5p,hsa-miR-4732-5p, hsa-miR-150, hsa-miR-424*, hsa-miR-4417, hsa-miR-4521, hsa-miR-365*, and hsa-miR-181a*.

3. The method of claim 1, comprising further determining in a sample from said subject the expression of one or more microRNAs selected from the group consisting of hsa-miR-617, hsa-miR-885-5p, hsa-miR-652, hsa-miR-1281, hsa-miR-4310, hsamiR-4800-3p, hsa-miR-4727-3p and hsa-miR-4791, in which a decrease in the expression of one or more of these microRNAs in the sample compared to the controls indicates that the subject suffers from or is likely to suffer from acne.

4. The method of claim 1, comprising further determining in a sample from said subject the expression of one or more microRNAs selected from the group consisting of hsa-miR-505*, hsa-miR-3651, hsa-miR-543, hsa-miR-23a*, hsa-miR-18a, hsamiR-1246, hsa-miR-3615, hsa-miR-4484, hsa-miR-4800-5p, hsa-miR-629*, hsa-miR-30e*, hsamiR-24-2*, hsa-miR-18b, hsa-miR-4667-5p, hsa-miR-483-5p, hsa-miR-27b*, hsa-miR-629, hsa-miR-187, hsa-miR-487a, hsa-miR-1244, hsa-miR-132, hsa-miR-550a*, hsa-miR-4286, hsa-miR-542-5p, hsa-let-7i*, hsa-miR-625, hsa-miR-411, hsa-miR-3175, hsa-miR-192, hsa-miR-503, hsa-miR-31, hsa-miR-1301 hsa-miR-92a-1*, hsa-miR-501-5p, hsa-miR-4732-5p, hsa-miR-150, hsa-miR-424*, hsa-miR-4417, hsa-miR-4521, hsa-miR-365*, hsa-miR-181a*, hsa-miR-155, hsa-miR-27a*, hsa-miR-941, hsa-miR-146b-3p, hsa-miR-146b-5p, hsa-miR-29b-1*, hsa-miR-421, hsa-miR-21, hsa-miR-212, hsa-miR-21*, and hsa-miR-223.

5. Use of the following microRNAs: hsa-miR-223, hsa-miR-21*, hsa-miR-212, hsa-miR-21, hsa-miR-421, hsa-miR-29b-1*, hsa-miR-146b-5p, hsa-miR-146b-3p, hsa-miR-941, hsa-miR-27a*, and hsa-miR-155, as biomarkers for acne, by detection in a dermal and/or epidermal sample.

6. A method for monitoring or determining the efficacy of an acne treatment in a subject comprising determining in a dermal and/or epidermal sample from said subject the expression of the following microRNAs: hsa-miR-223, hsa-miR-21*, hsa-miR-212, hsa-miR-21, hsa-miR-421, hsa-miR-29b-1*, hsa-miR-146b-5p, hsa-miR-146b-3p, hsa-miR-941, hsa-miR-27a*, and hsa-miR-155, and the decrease or increase in the expression of said microRNAs at the end of or during the treatment indicating the efficacy of the treatment.

7. Method according to one of claims 1-4, 6 and use according to claim 5, wherein the sample is a skin biopsy taken from affected skin areas.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **CUNLIFFE W.J.** *J. Dermatol. Treat.,* 2000, vol. 11 (2), S13-S14

- **BENJAMINI ; HOCHBERG.** *Journal of the Royal Statistical Society. Series B (Methodological),* 1995, vol. 57 (1), 289-300